# EUROPEAN PATENT APPLICATION

(11) **EP 4 108 081 A1**
(43) Date of publication of application: **28.12.2022**
(21) Application number: 22153789.7
(22) Date of filing: 27.01.2022
(51) Int. Cl.: A01K 67/033, C05F 17/05, B65D 21/08, B65D 25/04, B65D 81/38, B65D 85/50

(54) **MULTIFUNCTIONAL MODULAR UNIT FOR BREEDING OF INSECTS AND FOR BIOTREATMENT OF AGRIFOOD BY-PRODUCT AND/OR ORGANIC WASTE AND RELATED TECHNOLOGICAL SYSTEMS FOR ELECTROMECHANICAL STORAGE AND HANDLING**

(30) Priority: 28.01.2021 IT 202100001700
(71) Applicant: Econher S.r.l., 70010 Valenzano (BA) (IT)
(72) Inventor: Caramia, Giuseppe, 70043 Monopoli (BA) (IT); Caprio, Francesco, 70010 Valenzano (BA) (IT); Carucci, Vito, 70043 Monopoli (BA) (IT); Garganese, Gesumino, 70043 Monopoli (BA) (IT)
(74) Representative: De Tullio, Michele Elio

(57) **Abstract**

The object according to the present invention comprises a plurifunctional modular unit formed of several components assembled with each other as a function of the breeding and/or agri-food waste and/or organic waste biotreatment process.

Said modular unit features wide compatibility, in terms of size and morphology, with the ISO 6780:2003 international standard, consequently it can be used in pre-arranged and specialized technological storage and electromechanical handling systems or in standard industrial logistic plants, once properly modified and implemented with further technologies as necessary for said breeding and biotreatment activities.

The components of this plurifunctional modular unit, combined with each other, enable it to perform adult insect breeding and/or bioconversion/biostabilization and/or reproduction and egg laying activities in a controlled and accurate manner, each unit being a stand-alone one and treated as such even when grouped in multiple units for performing the same activities.

This invention also discloses some technological systems for storing and electromechanically handling single or multiple plurifunctional modular units essentially based on self-supporting structures provided with specific devices.

## Description

The present invention relates to the field of insect breeding and technical use thereof in waste biotreatment processes.

More specifically, the present invention provides a plurifunctional modular device for insect breeding and biotreatment, i.e. insect-mediated biostabilization and bioconversion of agri-food waste and/or organic waste to be cyclically treated; this modular device features wide compatibility with the present international standards and with some technological storage and electromechanical handling systems.

### Present status of the art

By biostabilization we mean a process that allows to biologically treat the putrescible fraction of waste, so as to make it inert and consequently less harmful for environment; in other terms, it is a waste treatment operation that consists of producing a stabilized organic fraction starting from a wet fraction.

Bioconversion is an innovative process that takes advantage of the capacity some saprophagous organisms, for example insects, have of converting organic waste into a biomass; this process takes advantage of the larval stage of some saprophagous dipterans, in particular *Hermetia illucens,* which are capable of growing very rapidly by digesting organic waste, and accumulating fats and proteins, without representing any risk for humans.

Bioconverting organic residues by use of larvae makes it possible to reduce food wastage and to treat greater and greater quantities of waste, makes it possible to find alternative and low-cost sources for producing zootechnical feeds, cosmetics, polymers, and energy, and also makes it possible to reduce costs for those companies that have to dispose organic waste which, once bio-converted, makes up fertilizers featuring a high agronomical value.

Plants and devices for insect breeding usable in organic waste biotreatment processes are known in the present status of the art; industrial plants having different characteristics as a function of the quantity and type of agri-food waste and/or organic waste to be treated by means of saprophagous insects are also known.

In particular, several patent documents are known in the present status of the art associated with the inventive idea developed in the present patent application.

Patent document US2019191678 discloses a system and method for breeding flies, specifically Black Soldier Flies (BSFs) that can be used for bioconversion of organic waste into value added products such as fertilizers and animal feed. Particularly, the proposed system and method facilitate in breeding Black Soldier Flies (BSFs) and harvesting their larvae in a large scale.

Patent document US10842138 provides methods, devices and systems for cultivating insect larvae and processing organic material. Exemplary systems and methods provide an arrangement of shelves containing trays for larvae cultivation and frass collection. The shelves are maintained in a controlled environment and are adapted to receive a feedstock comprising organic material for conversion into insect biomass. Patent document CN110014017 discloses a kitchen waste bioconversion system comprising a pre-treatment system used for separating kitchen waste and obtaining solid organic residues, a biological treatment system that takes these solid organic residues as a raw material of a growing medium for breeding insects; and a fermentation system used for fermenting the manure obtained in the insect reproduction process; the exhaust gases and the waste waters generated in the whole process are effectively collected and treated, so as to prevent any influences onto the surrounding environment and limit secondary pollution. After being submitted to a biological treatment, kitchen waste is converted into bioproteins and organic fertilizer, so that the economical added value is high, and profits are further increased.

The continual evolution of goods storage and electromechanical handling technology offers materials and products to humans that are more and more innovative and capable of providing performances that can be taken advantage of in the everyday life. Many efforts have been made over time to be able to get a standardization in goods storage and handling systems, in order to replace the most ancient storage systems, such as, for example, wooden boxes and barrels.

At present, the most popular goods storage and electromechanical handling system is based on the use of so-called pallets.

A pallet is a flat structure on which goods are placed, so as to form a loading unit that allows its handling and storage by using means like forklifts, pallet trucks, and the like. As easily imaginable, pallet sizes are extremely variable, in order to be able to meet the most various application requirements; such variety of sizes forced final users to create a standardization.

The initial adoption of an arbitrary method was particularly penalizing for those who used them, because every pallet could not be interchanged, but used by its owner only; the uniformity adopted today makes it possible to interchange platforms and speed-up the system.

Thanks to the intervention of the International Organization for Standardization (ISO), the standard sizes of the pallets have been set up in ISO Standard 6780:2003, subsequently updated many years later, namely in 2014.

An object of the present invention is to provide a plurifunctional modular device for insect breeding and biotreatment, i.e. biostabilization and bioconversion, of agri-food waste and/or organic waste, which features wide compatibility with the present international standards and some specific storage and electromechanical handling technological systems (ISO 6780:2003).

A further object of the present invention is to provide a plurifunctional modular unit that allows to perform breeding and/or bioconversion/biostabilization activities in a controlled and accurate manner, said unit being a stand-alone one and treated as such even when grouped together with others to form multiple units.

Another object of the present invention is to provide cyclical or non-cyclical procedures, functional to the insect breeding and/or organic waste bioconversion and unsorted waste biostabilization processes; said procedures can be performed according to a scheduling provided by an operator or based on changes in the environmental conditions as detected by an appropriate set of sensors.

The insect breeding and agri-food waste and/or organic waste biotreatment modular unit according to the present invention also makes it possible to isolate and render every system that it is part of, climatically autonomous.

### Summary of the invention

The object of the present invention comprises a plurifunctional modular unit comprising several components assembled with each other as a function of the breeding and/or agri-food and/or organic waste biotreatment process.

Said modular unit features a wide compatibility with the ISO 6780:2003 international standard in terms of size and morphology, hence it can be used in pre-arranged and specialized storage and electromechanical handling technological systems or in standard industrial logistic plants, once properly modified and implemented with further technologies as necessary for said breeding and bioconversion activities.

The components of a plurifunctional modular unit according to the present invention, as detailed below, once combined with each other, make it possible for it to perform breeding and/or bioconversion/biostabilization and/or adult insect reproduction and egg laying activities in a controlled and accurate manner, each unit being a stand-alone one and treated as such even if grouped into multiple units for performing identical activities.

Specifically, this modular unit solves a major technical problem: it remarkably reduces the volume of air to be treated and, above all, prevents the volatile components (Volatile Organic Compounds and Volatile Inorganic Compound, VOC and VIC respectively) that are generated by vaporization during a bioconversion process, from reaching the components of a plant, thus reducing the need for using materials specifically resistant to such compounds.

Another huge advantage resulting from the punctual control of the individual plurifunctional unit is in that the amount of latent heat emitted by a waste matrix being bioconverted during a bioconversion cycle, varies with the amount of waste that is oxidized during the process; therefore, this advantage will result from the possibility of recovering latent heat from those units which emit more heat and, conversely, addressing it to those units which need for it for the wellness of the insects internally thereto.

This invention also discloses some storage and electromechanical handling technological systems for individual or multiple plurifunctional modular units basically based on self-supporting structures equipped with specific devices.

### Brief description of the figures

The device according to the present invention is described here below with reference to some preferred, but not exclusive, embodiments, which are illustrated for explanatory, not limitative purposes only, and with a special reference to the attached figures, wherein:
- figure 1 is an axonometric view of a modular unit functional to the breeding and bioconversion and/or biostabilization processes to be connected to external thermal and electric power supply plants;
- figure 2 is an axonometric view of a modular unit functional to the breeding and autonomous bioconversion and/or biostabilization processes, both for electric power supply and for thermal conditioning;
- figure 3 is an axonometric view of the modular unit of figures 1 and 2 in a pre-assembling step with an interchangeable cover;
- figure 4 is an axonometric view of a modular unit functional to the adult insect breeding and egg laying processes to be connected to external thermal and electric power supply plants;
- figure 5 is an axonometric view of a modular unit functional to adult insect breeding and autonomous egg laying processes, both for electric power supply and thermal conditioning;
- figure 6 is an axonometric view of the modular unit of figures 4 and 5 in a pre-assembling step with an interchangeable cover;
- figure 7 is a top axonometric view of a base component common to all modular units;
- figure 8 is a bottom axonometric view of the base component of figure 7;
- figure 9 is a top axonometric view of the component functional to the breeding and/or bioconversion and/or biostabilization processes present in the modular units of figures 1 and 2;
- figure 10 is a bottom axonometric view of the component of figure 9;
- figure 11 is a top axonometric view of the cover of the component functional to the breeding and/or bioconversion and/or biostabilization and/or egg laying processes present in the modular units of figures 1 and 4;
- figure 12 is a bottom axonometric view of the cover component of figure 11;
- figure 13 is a top axonometric view of the adult insect breeding and egg laying cell component present in the modular units of figures 4 and 5;
- figure 14 is a bottom axonometric view of the cell component of figure 13;
- figure 15 is a top axonometric view of the cover of the component functional to the breeding and/or bioconversion and/or biostabilization and/or egg laying processes present in the modular units of figures 2 and 5;
- figure 16 is a bottom axonometric view of the cover component of figure 15;
- figure 17 is an axonometric view of a modular unit functional to the breeding and bioconversion and/or biostabilization and/or egg laying processes whose cover is stably connected to the storage systems;
- figure 18 is an axonometric view of the modular unit of figure 17 in a pre-assembling step;
- figure 19 is a top axonometric view of the cover of the component functional to the breeding and bioconversion and/or biostabilization and/or egg laying processes present in the modular unit of figures 17 and 18;
- figure 20 is a bottom axonometric view of the cover of figure 19;
- figure 21 is an axonometric view of a first storage and handling electromechanical handling system of plurifunctional modular units;
- figure 22 is an axonometric view of a second storage and electromechanical handling system of plurifunctional modular units;
- figure 23 is an axonometric view of a third storage and electromechanical handling system of plurifunctional modular units;
- figure 24 is an axonometric view of a fourth storage and electromechanical handling system of plurifunctional modular units;
- figure 25 is an axonometric view of a casing applied to the storage and electromechanical handling system of plurifunctional modular units of figure 22.

### Detailed description of the invention

The present invention discloses several plurifunctional modular units for insect breeding and biological treatment of agri-food waste and/or organic waste and/or biostabilization thereof.

Every modular unit comprises several components which, assembled with each other, make it possible to perform a number of functions, such as, for example, insect breeding and/or bioconversion of organic waste.

The modular units feature a wide compatibility with the ISO 6780:2003 international standard in terms of size and morphology, consequently they can be used in storage and electromechanical handling technological systems or in standard industrial logistic plants, once properly modified and implemented with further technologies as necessary for said breeding and bioconversion activities to take place.

All elements detailed below, combined with each other, make it possible for each individual modular unit thus implemented to perform breeding and/or agri-food waste and/or organic waste biotreatment activities in a controlled and accurate manner, the modular units being stand-alone ones and treated as such even when grouped together into multiple units for one and the same activity.

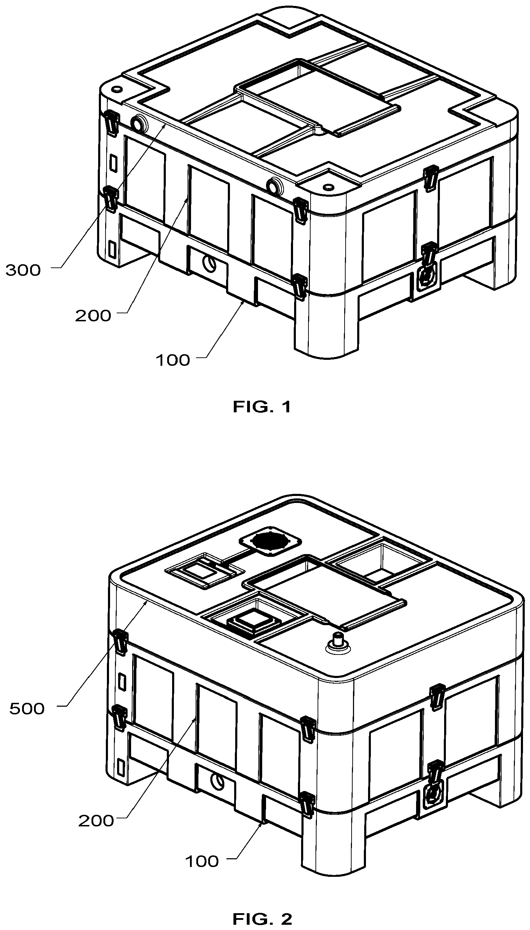

Figures 1, 2, and 3 show axonometric views of modular units functional to the breeding and/or bioconversion and/or biostabilization processes respectively assembled.

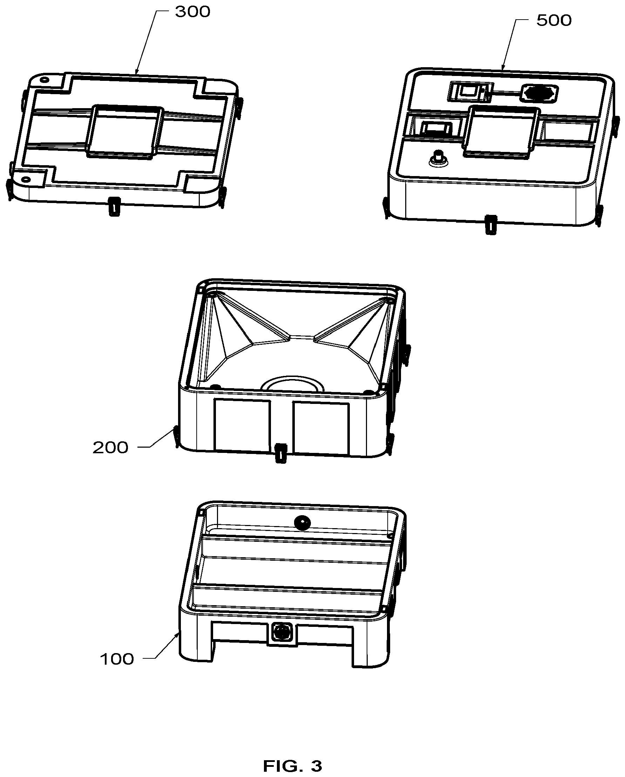

Figure 1 shows a functional system to be connected to external thermal and electric power supply plants; figure 2 shows a functional system that is autonomous both in terms of electric power supply and thermal conditioning; figure 3 shows how both systems are pre-assembled.

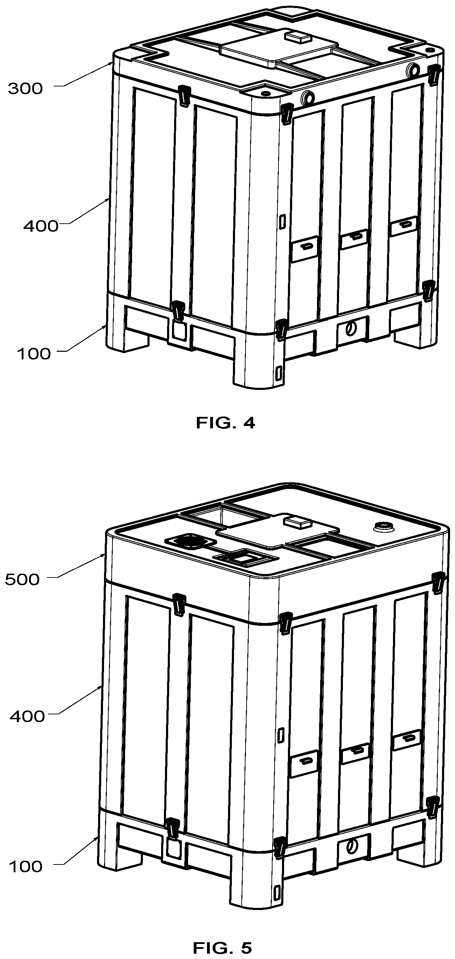

Figures 4, 5, and 6 show axonometric views of modular units functional to the adult insect breeding and egg laying processes, being assembled and pre-assembled respectively.

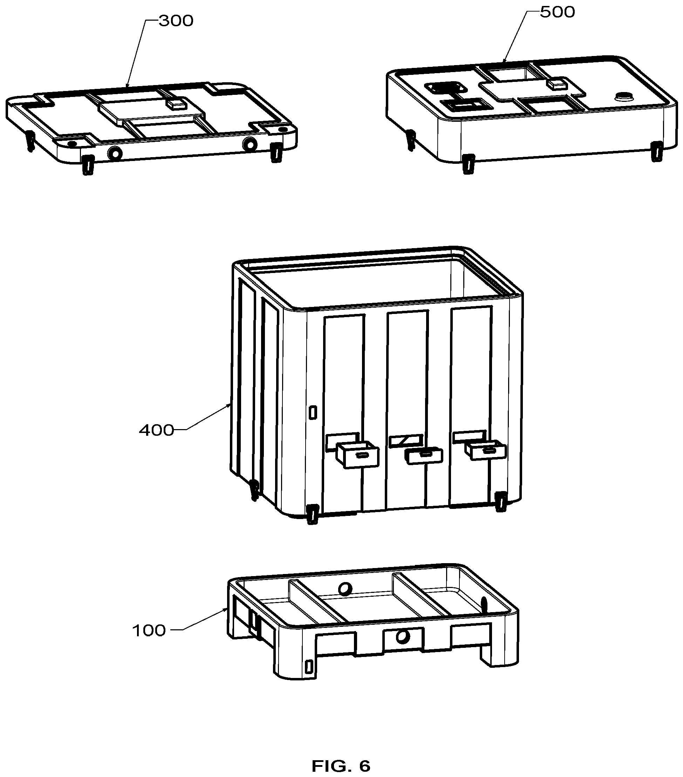

Figure 4 shows a functional system to be connected to external thermal and electric power supply plants; figure 5 shows a functional system that is autonomous for both electric power supply and thermal conditioning; figure 6 shows both systems being pre-assembled.

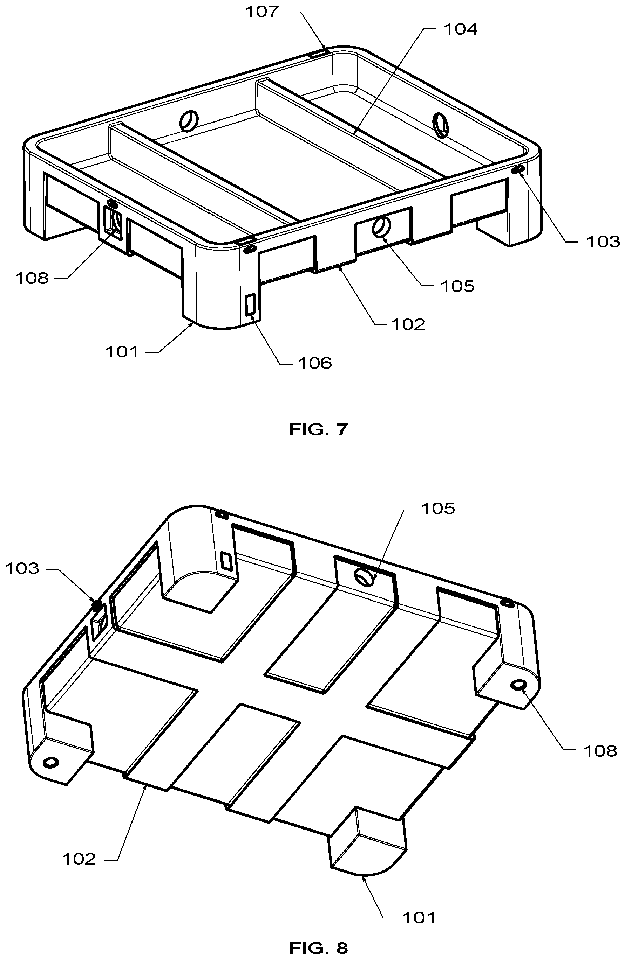

Figures 7 and 8 show a component 100 that makes up the base component common to all modular units, as well as an interface to the handling systems used to handle the same units through the storage and electromechanical handling systems that will be described later.

The base component 100, provided with an RFID, comprises a parallelepiped-shaped casing whose upper face is open, having dimensions in the horizontal plane compatible with the ISO 6780:2003 international standard and a height ranging from 200 to 600 mm.

Protrusions 101 located at the four corners of said component operate as feet, which facilitate their picking-up and placement in the storage and electromechanical handling systems; it is made from a double wall plastic material, with a total thickness ranging from 8 to 60 mm, which allows to form a gap to be possibly used for insulating the component itself through insufflation of an insulating material (preferably polyurethane foam).

This component is externally provided with ribs or stiffeners 102, as necessary to support the full load weight of components placed above, as well as with appropriate protrusions and/or arrangements 103 on the top, at the four sides, to allow for its coupling with a component placed above by means of appropriate joints and/or handles.

One or several ribs 104 partition the inner space into compartments and stiffen its structure; they are technical compartments, provided with exhaust holes 105 and/or holes for interfacing to the external world, functional to the breeding and/or bioconversion activities performed in the individual units.

Sliding electrical contacts 106 are inserted at the base of at least two feet, each in a diagonally opposed position, to supply power to the complete modular unit when it is installed inside the storage and electromechanical handling systems.

An equal number of electrical contacts 107 are equipped on the upper edge of the component 100, in correspondence with and directly connected to the electrical contacts 106, to transfer an electric current to the component placed above.

In another possible configuration, the remaining two feet are provided with cylindrical cavities 108, which enable said component to put a capillary climatization plant, if any, in communication with the individual plurifunctional units, should the latter be installed in the station inside the storage and electromechanical handling systems.

Said cavities are properly equipped with normally closed check valves and are implemented in such a way that connectors properly preset in the storage and electromechanical handling system can cross it; these connectors are implemented in such a way that, whenever the foot of a component rests in the corresponding parking point in the storage system, a connection is established to the air distribution or return network of said climatization plant via the mechanical opening of the above-described check valve.

The communication with the climatization system implemented by this connector continues internally to the foot via an appropriate cavity which terminates, in the surface of the component 100, in a hole provided with a further edge capable of putting said cavity of the component 100 in communication with its corresponding cavity (cavities) in the component 200 described below.

Finally, through holes 109 are present on the lateral surface of the component 100, used to create an oxygen gradient between the space internal to the component 200 and the outside of the plurifunctional component.

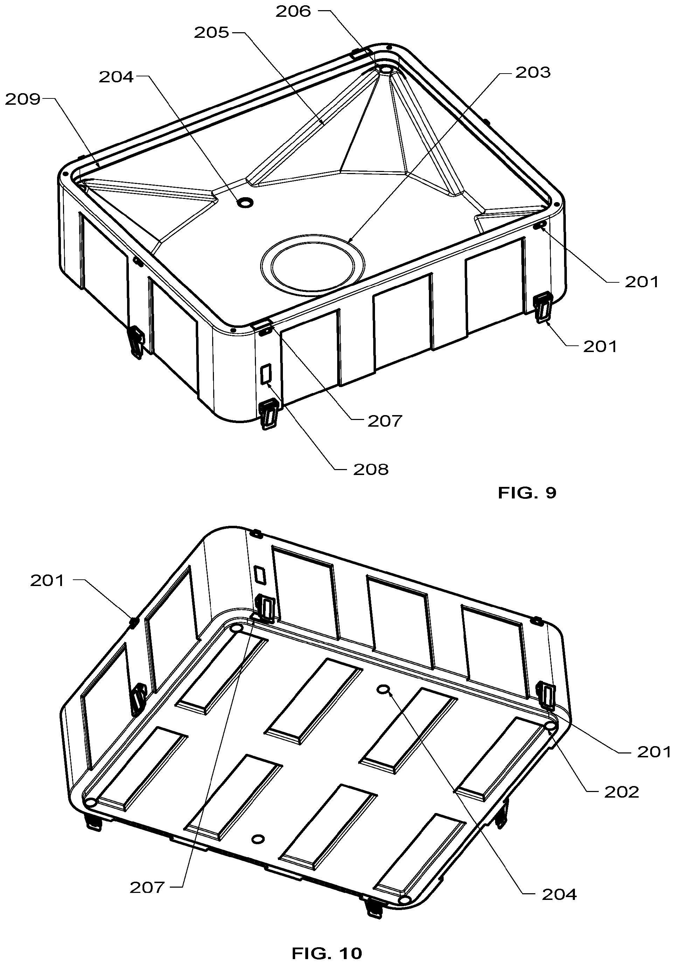

Figures 9 and 10 show the component 200, which represents a component functional to the breeding and/or bioconversion processes.

Said component 200 comprises a parallelepiped-shaped container whose upper face is open, has a height in the range from 250 to 550 mm and dimensions in the horizontal plane compatible with the ISO 6780:2003 international standard.

The component 200 is positioned above the base component 100 (having equal dimensions in the horizontal plane) in order for it to be handled.

The component 200 is provide with RFID, as well as with appropriate protrusions and/or arrangements 201 on the top and on the bottom in correspondence with the four sides, in order to make it possible its coupling with the component placed above and/or below, by using appropriate joints and/or handles.

In a possible configuration, the component 200 is provided with one or several holes 206 connected to cavities implemented in such a way as to communicate with their corresponding tops of the cavities described in the component 100, on the lower surface of the component 200, and specifically in the point 202, which are put in communication with the contact surface of the component 300, via appropriate cylindrical connectors not shown in the figures, and in this configuration can convey climatization to the component 300.

The inner bottom plane of the component 200 comprises a central circular area 203, 2 to 15 mm depressed with respect to the point where the bottom itself joins to the sides of the parallelepiped.

The component 200 also comprises several holes 204 close to the walls for allowing percolation of any liquids in excess present in the breeding matrix or to be bioconverted/biostabi lized.

Whenever the species of insect reared or used for bioconversion/biostabilization requires it, the inner walls of the component 200 are provided with migration paths, physically consisting of protrusions of trapezoidal shape 205, placed all along the inner walls at a regular distance variable from 5 to 10 cm from each other, and comprising a hole 206 (migration hole) at their tops, at the middle of the higher side of the trapezium. The holes 202 and 206 connect the inner space of the component 200 to the component 100 via appropriate connectors, not shown in the figure, or empty gaps specifically arranged in the component 200, and correspond to appropriate empties spaces arranged in the component 100.

Sliding electrical contact 207, connected to each other, are inserted on the lower and upper edges of the component 200, each in a diagonally opposed position, to transfer electric current to components placed below and above.

As an alternative to the electrical contacts 106 inserted in the component 100, sliding electrical contacts 208 are inserted on two sides of the component 200, each in a diagonally opposed position and in correspondence with the contacts 207, to supply power to the units whenever they are installed inside the storage and electromechanical handling systems.

Metal tracks 209, parallel to each other, made from a conductor material and spaced by 2 to 5 mm from each other, are present at the top of the inner walls of the component 200, close to the upper edge and in any case above the migration holes, if any; electricity can be conveyed on said tracks, propagated by the component 100 or alternatively by the components 200, 500 or 600 (see below), in the form of a direct current at a voltage variable from 24 to 48 Volts, depending on the application, and a current variable from 0 to 4 Amperes in order to prevent insects from moving away from the component 200 in an uncontrolled manner.

The component 200 is made from a double wall non-toxic food plastic material (preferably high-density polyethylene) having a total thickness in the range from 8 to 60 mm, which allows to internally form a gap to be possibly used for insulating the component itself through insufflation of an insulating material.

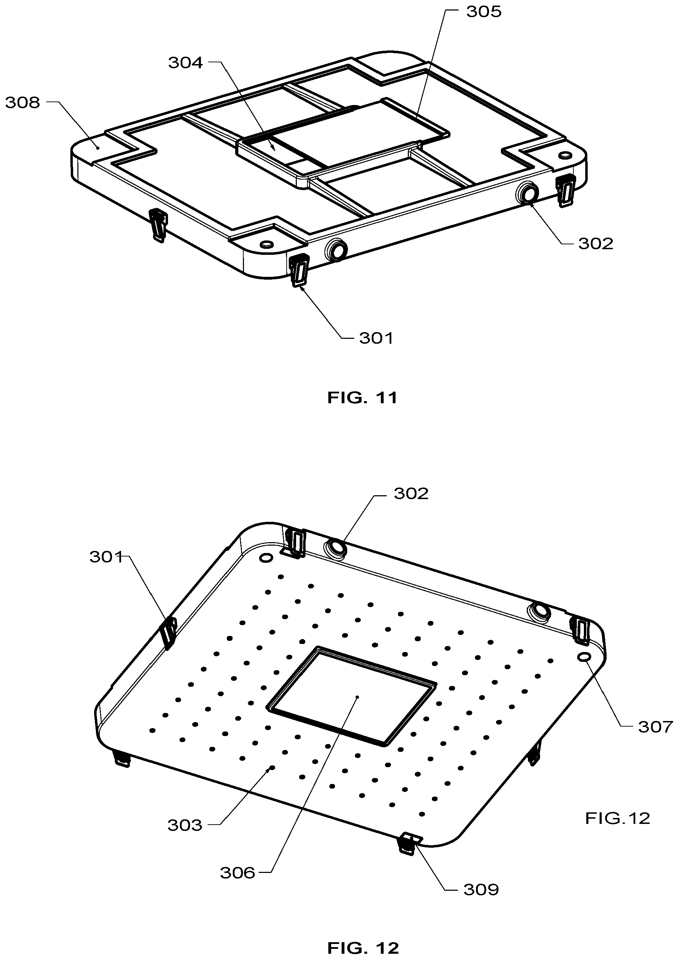

Figures 11 and 12 show the component 300, which operates as a cover for the component 200 functional to the breeding and/or bioconversion processes, and/or for the component 400 functional to the adult insect breeding and egg laying processes. Said component 300 is used for closing the components 200 and 400, which it is connected to, by means of appropriate external locking joints and/or handles 301. The cover component 300 comprises a parallelepiped monobloc having dimensions in the horizontal plane compatible with the ISO 6780:2003 international standard and a height in the range from 100 to 300 mm.

It is formed from a double wall non-toxic food packaging plastic material, with a total thickness in the range from 8 to 60 mm, which allows to form a gap to be possibly used for insulating the component itself by insufflation of an insulating material. Connection holes 302 are positioned on the lateral surface to connect to the climatization plant; said connection holes are implemented in such a way as to be connected to two cavities present in the thickness of the component 300, parallel to each other and placed along the shorter sides of the component itself, and said cavities are connected to at least one hollow space, orthogonal to said cavities and parallel to the longer side of the component 300 (major axis), and provided with at least one hole having a minimum diameter of 0.5 cm on its development along said axis and on its lower surface, which puts said cavities in communication with the inside of the component 200 with at least one distribution hole 303 present on the lower surface of the component 300.

In alternative to the connection holes 302, at least two connection holes 307 are inserted (to connect to the climatization plant), characterized in that these are implemented on the lower surface of the component 300 and are provided with a normally closed check valve, in correspondence with at least two of the migration holes possibly arranged in one of the possible embodiments of the component 200; said connection holes are also implemented in such a way as to be connected to two cavities present in the thickness of the component 300, parallel to each other and placed along the shorter sides of the component itself, and said cavities are connected to at least one hollow space, orthogonal to said cavities and parallel to the longer side of the component 300 (major axis), and provided with at least one hole having a minimum diameter of 0.5 cm on its development along said axis and on its lower surface, which puts said cavities in communication with the inside of the component 200.

The cover component 300 is implemented in such a way as to benefit from an opening 304 which, during the breeding and/or bioconversion/biostabilization process, is used to load a batch of organic matrix to be bioconverted/biostabilized or to be administered as a nourishment for the reared insects and is provided with a hatch 305 having an electromechanical opening.

For the adult insect breeding and egg laying process, the opening 304 is used to accommodate a lighting system 306, preferable a LED one.

A depression 308, present at every upper corner of the component 300, operates as an accommodation and lead-in facility for any plurifunctional modular units stacked thereonto, which can be taken advantage of in the case of a stacking storage. Sliding electrical contacts 309 are inserted on the lower edge of the component 300, each in a diagonally opposed position, to receive an electric current from a component placed below.

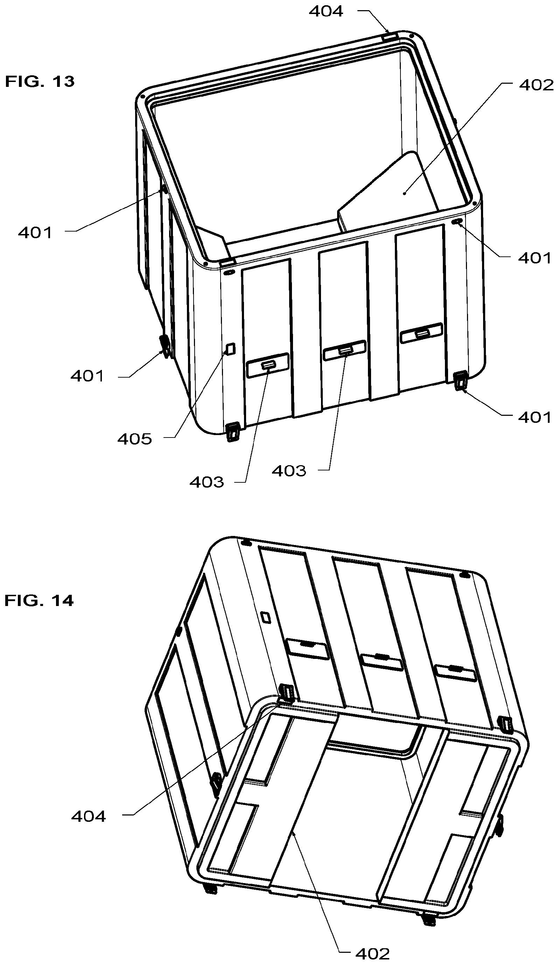

Figures 13 and 14 show the component 400 that represents the cell component for adult insect breeding and egg laying and comprises a parallelepiped-shaped container, the upper face of which is provided with a removable frame, not shown, provided in turn with a metal or plastic wire net, with meshes preferably having dimensions 0.9 x 0.9 mm.

Said cell component 400 has dimensions in the horizontal plane compatible with the ISO 6780:2003 international standard and a height in the range from 700 mm to 2000 mm, preferably 1000 mm.

The component 400 is positioned above the component 100 (having equal dimensions in the horizontal plane) and is provided with appropriate protrusions and/or arrangements 401 on the top and on the bottom at the four sides to make it possible for it to couple with a component placed above and/or below by means of appropriate joints and/or handles.

The bottom plane of the component 400 comprises two opposed latero-medially tilted planes 402, at an angle of preferably 60°, which join the lateral surfaces of the component 400 to the ribs 104 present in the component 100.

No continuity exists between the two opposed planes, consequently the bottom plane includes a hole which allows the space internal to the component 400 to communicate with that present between the ribs of the component 100, whenever this one is placed below it, and its function is to convey the dead insects from the component 400 to the component 100, taken out from it through a suction system via holes 105.

The cell component 400 is provided with RFID and can be made from a double wall plastic material, with a total thickness ranging from 8 to 60 mm, which allows to form a gap to be possibly used for insulating the component itself by means of insufflation of an insulating material.

Alternatively, the lateral walls of the component 400 can comprise frames provided with a metal wire net with meshes of 0.9x0.9 mm connected to each other.

In a further possible embodiment, the side walls of the component 400 comprise frames provided with panels made from a transparent to light material connected to each other.

In all possible embodiments, at least one of the side walls of the component 400 is provided with three compartments 403 accessible from the external world.

Sliding electrical contacts 404 connected to each other are inserted on the lower and upper edges of the component 400, each in a diagonally opposed position, to transfer an electric current to components placed below and above.

As an alternative to the electrical contacts 106 inserted in the component 100, sliding electrical contacts 405 are inserted on two sides of a tank, each in a diagonally opposed position and in correspondence with the contacts 404, to supply power to the units whenever they are placed inside the storage and electromechanical handling systems.

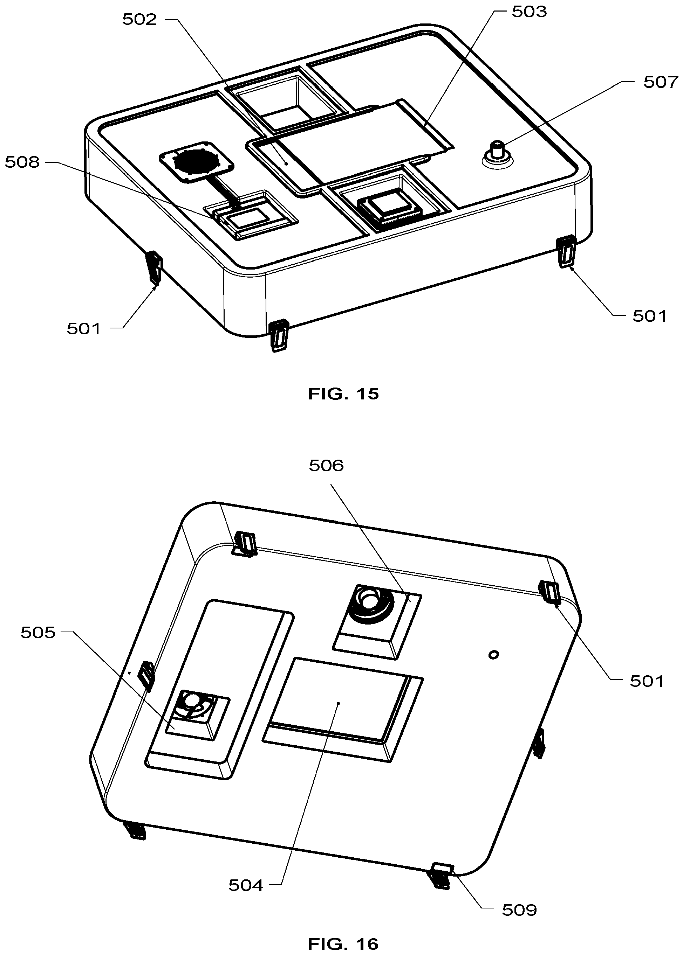

Figures 15 and 16 show the component 500 which operates as a cover for the component 200, functional to the breeding and/or bioconversion/biostabilization processes, or for the component 400, functional to the adult insect breeding and egglaying processes.

Said component 500 is used for closing the components 200 and 400 it is connected to by means of appropriate external locking joints and/or handles 501.

The cover component 500 comprises a parallelepiped-shaped monobloc having dimensions in the horizontal plane compatible with the ISO 6780:2003 international standard and a height in the range from 100 to 300 mm and is made from a double wall non-toxic food packaging plastic material, with a total thickness ranging from 8 to 60 mm, which allows to form a gap to be possibly used for insulating the component itself by insufflation of an insulating material.

The cover component 500 is implemented in such a way as to benefit from an opening 502, the function of which is to allow a batch of organic matrix to be bioconverted/biostabilized or to be administered as a nourishment for the reared insects to pass through for the breeding and/or bioconversion/biostabilization process, and is provided with a hatch 503 complete with an electromechanical opening facility. In the adult insect breeding and egg laying process, the opening 502 is used to accommodate a lighting system 504, preferably a LED one.

The cover 500 is implemented in such a way as to provide, internally thereto, appropriate compartments for containing an ambient humidification system, an internal air extraction fan connected to an appropriate passage 505 for letting the exhausted air go out via a technical filter, a second opening for injecting external air 506; both passages are characterized in that they are connected to a container, via separate paths, where specific devices are accommodated and receive electric power for heating or cooling the air that crosses said container, preferably consisting of one or several Peltier cells.

The component 500 might also be provided with a wi-fi antenna 507 or an antenna according to another similar technology for data transmission, a set of sensors for controlling humidity, temperature, oxygen, etc.; a programmable logic controller (PLC) for controlling the above-described devices; an RFID tracking system; a battery 508 for said devices, as necessary when the modular unit is placed outside the storage and electromechanical handling system.

Sliding electrical contact 509 are inserted on the lower edge of the component 500, each in a diagonally opposed position, to receive an electric current from a component placed below.

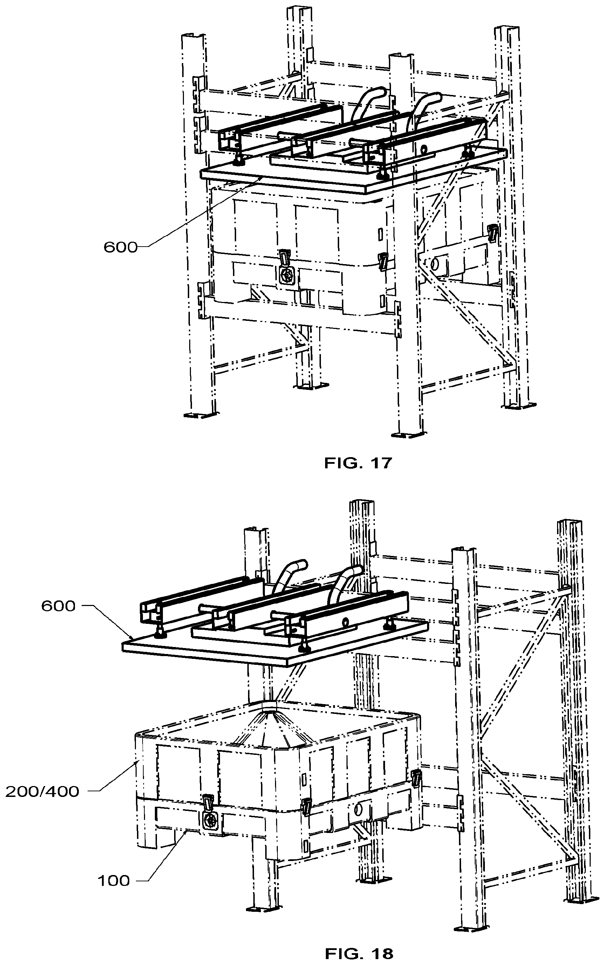

Figures 17 and 18 show axonometric views of a modular unit functional to the breeding and bioconversion and/or biostabilization and/or egg laying processes, whose cover is stably connected to the storage system, being assembled and pre-assembled respectively.

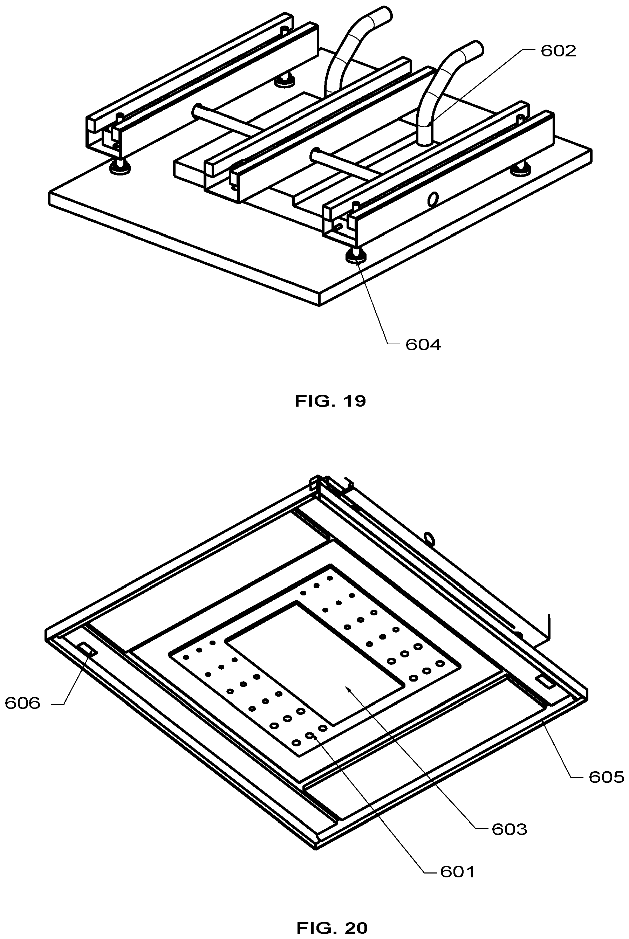

Figures 19 and 20 show a component 600 which operates as a cover component to close one or several plurifunctional modular units; as a matter of fact, the component 600 can be alternatively joined to the components 200 or 400.

The component 600 can be used to convey the climatization, via holes 601 joined to appropriate sets of pipes 602, as well as to convey lighting, if any, implemented by means of appropriate lamps 603, preferably LED lamps.

Said cover 600 is connected to the storage and electromechanical handling systems, via preferably pneumatic joints 604, which enable the component itself to move away by few centimetres (downward) from the horizontal structures arranged in the storage systems, as soon as one or several plurifunctional modular units reach the destined position below said component 600.

The lower portion of the component 600 also comprises a gasket all along the edge 605, or several gaskets in correspondence with several units, should the component 600 be used for a multiple closing of several units, in order to provide for a perfect sealing between the component itself and the modular unit(s).

The component 600 also comprises one sliding electrical contact 606, which mates a corresponding component 200 or 400 upon placing the latter in the storage system of the modular unit (as described below), the function of which is to supply electric power to the modular unit.

The function performed by the pneumatic mechanism here described is to seal the component(s) 200 or 400, in order to prevent any exhalations produced in said components from propagating to the storage system during the breeding, bioconversion, and biostabilization processes; these exhalations can be collected and possibly used for extracting ammonium compounds and/or for reducing the particulate matters and/or volatile inorganic compounds (VIC) and volatile organic compounds (VOC) possibly generated by the process itself, before they reach the external environment outside the storage system.

The control of the adult insect breeding and/or egg laying and/or organic waste bioconversion and unsorted waste biostabilization processes according to the present invention features cyclical or non-cyclical procedures, functional to said processes, which can be performed according to a scheduling provided by an operator or as a function of changes taking place in the environmental conditions, as detected by appropriate set of sensors and controlled by appropriate hardware/software devices. Every hardware/software device comprises a central check and control unit and several peripherals connected thereto, capable of detecting temperature, quantity of oxygen, humidity, and pH internally to the components 200 and 400 of the plurifunctional modular units; said peripherals can be installed alternatively in the cover components 300, 500, 600 of the modular unit.

The task of a hardware/software device is thus to detect said parameters, store them on a dedicated server, as well as to send appropriate controls, aiming at controlling the processes by means of dedicated actuators.

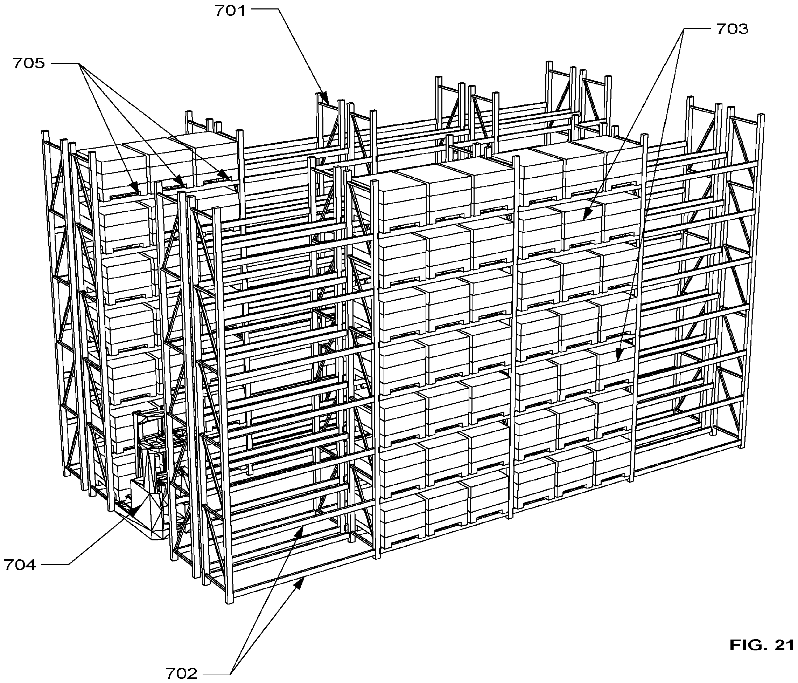

Figures 21 to 25 illustrate some technological systems 700 for storing and electromechanically handling plurifunctional modular units, essentially based on self-supporting structures provided with specific devices, which comprise single or multiple picking-up facilities depending on the size to be treated.

More specifically, figure 21 shows a system formed of vertical elements 701 of variable heights and depths (uprights and/or shoulders and/or sides frames), properly structured and possibly latticed, interconnected by means of girders 702, to form rows spaced away from each other to create appropriate corridors according to a project diagram adopted as a function of the actual size.

The system supports the formation of n fixed inter-storey loading levels 703, where n is a function of the height available and the maximum load elevation workable by the lifting and handling means 704 used.

In fact, said system makes it possible to handle the individual plurifunctional modular units in the corridors by means of stackers, front and retractable forklifts, as well as bilateral and trilateral, either operator-guided or automatically guided (e.g., automatic guided vehicles, laser guided vehicles, intelligent guided vehicles, or AGV, LGV, IGV respectively, etc.) forklifts provided with appropriate forks.

The system also supports the use of appropriate sliding and direct electrical contacts 705 for every position reserved for the plurifunctional modular units.

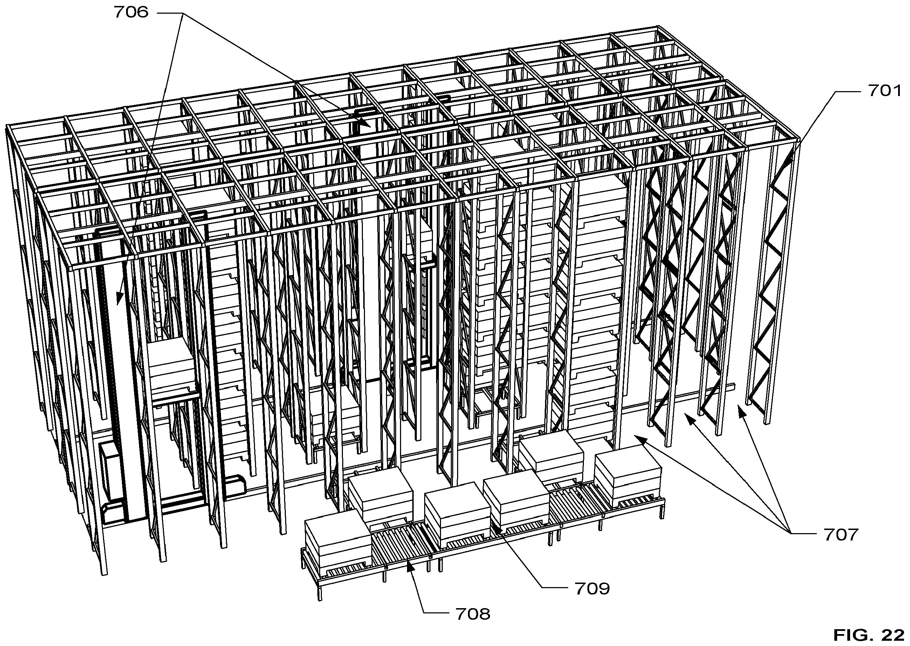

Figure 22 shows another storage and electromechanical handling system for plurifunctional modular units, formed of a plurality of vertical elements 701 featuring different heights and depths (uprights and/or shoulders and/or side frames), properly structured and possibly latticed and spaced away from each other as a function of the project diagram requirements, to form rows connected by a system of lattices and profiles and multiplied in depth to allow insertion of the handling systems 706 of the plurifunctional modular units (picked-up in a single or multiple mode) into the vertical compartments 707 thus created.

In particular, the vertical elements are provided with a number of brackets full-height mounted laterally thereto, equally spaced away by at least 50 mm from each other, so as to make it possible to take full advantage of the volumes internal to the same vertical compartments; as a matter of fact, if modular units of different heights are to be combined together in the vertical compartments, the volume available might be exploited at the best thanks to the positioning of the trays and/or frames containing the modular units on the preset brackets spaced away by at least 50 mm from each other. The automated three-axis handling systems 706 used in the system of figure 22 can be of a horizontal girder or vertical gantry or overhead crane or reversed translation type, all of them in any case provided with appropriate winches for picking-up and fast handling of the modular units toward appropriate catenaries/roller conveyors 708, which are used to transport them toward a manual, semi-automated or fully automated control station 709 used to control the modular units for loading, unloading, and/or cyclical control purposes.

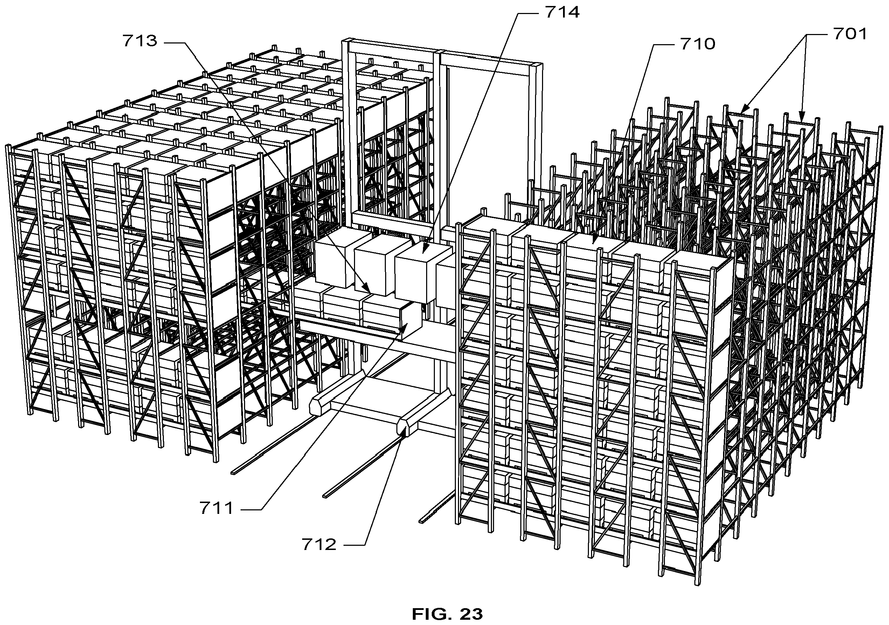

The storage and electromechanical handling system shown in figure 23 includes the same vertical elements as described for the system depicted in figure 22, the only difference being in that the brackets present in the vertical compartments are replaced by horizontal spacers and/or stringers, which determine positions at a fixed height, on which plurifunctional modular units 710, all featuring the same maximum dimension, can be arranged according to a multi-depth approach 710, being inserted onto appropriate latticed skids 711, to be handled by means of an appropriate automated machine 712.

The latter moves along an appropriate corridor to make it possible to remove the skids 711 laterally, thus making it possible to operate on a dual front, and consequently to handle a multiple quantity of modular units.

In the system depicted in figure 23, the handling machine 712 used to handle the skids 711 is possibly formed of a special trailer, having a minimum front dimension equal to at least that of the skid itself, such as to take said skid unit out with its multiple load of modular units and to perform some operations, in particular that of administering the matrices by means of an appropriate meter 713 mounted on the same trailer, which receives the matrices from one or several properly arranged buffer tanks 714 which the trailer 712 is provided with, on board the machine, without any further movement toward other work positions.

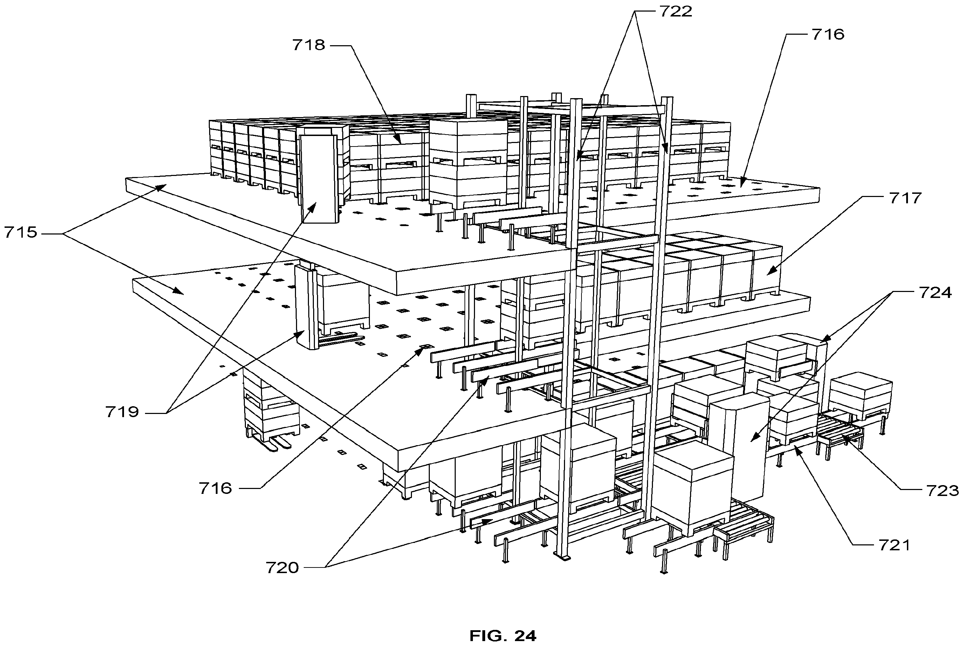

The storage and handling system of the plurifunctional modular units shown in figure 24 exploits wide horizontal surfaces 715 with vertical development capabilities.

This system needs multi-story structures (stacked galleries) implemented through use of wide bays, on which stations are inserted, according to a predetermined electrified grid 716, where the modular units are placed in a single mode 717 or in a stacked mode 718.

The modular units will be cyclically moved by one or several automatic guided machines 719 (AGV, LGV, IGV, etc.), which convey them to appropriate areas or conveyors 720 in order for them to be sorted toward the working, test, and/or production bays 721 by means of elevators 722 and further chain and/or roller conveyors 723, in order for them to be finally placed in the points of the grids 716 reserved therefor.

In the case of modular units arranged in a stacked mode 718, in order for them to reach the bay 721 and, after the appropriate cyclical handling operations, the successive points of the grid 716, a decoupling and subsequent coupling is necessary by using appropriate stacking machines 724.

In order for every storage and electromechanical handling system of plurifunctional modular units to be insulated and climatically autonomous, a casing is necessary to enclose it.

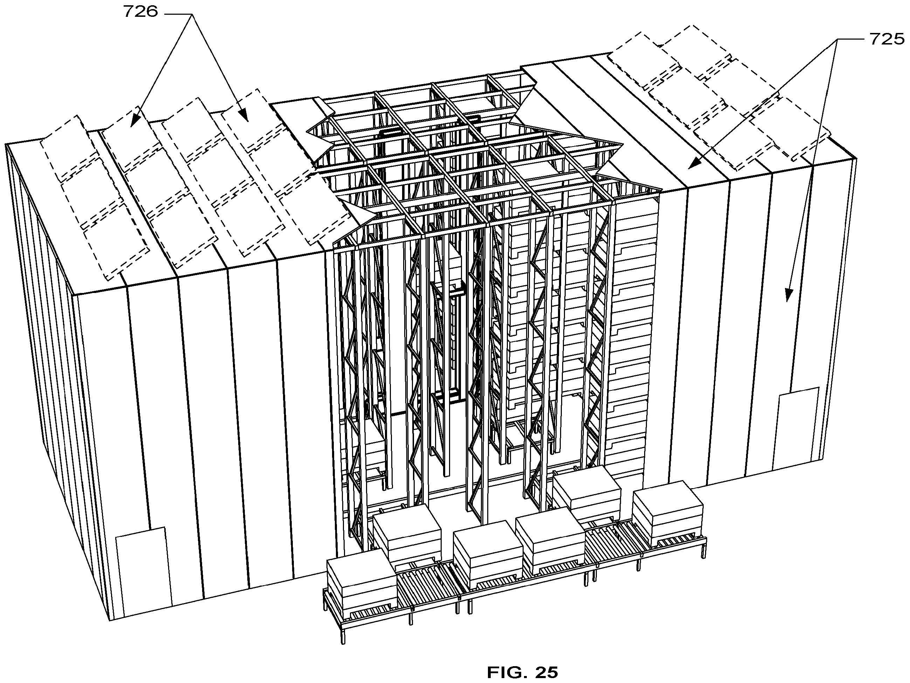

Figure 25 shows a casing applied to the storage and electromechanical handling system of the plurifunctional modular units of figure 22.

This casing comprises walls and peripheral coverings 725 of various natures, to be applied to the systems shown in figures 21 throw 24, so as to make them insulated from any types of structure and/or environment accommodating them and allowing their installation also outside industrial factories.

The covering and the walls are implemented by a set of insulating panels made from metal (or wood) and/or technical materials providing high thermal and acoustical insulation and mechanical strength; said panelling/coverings can be properly decorated on the outer side, in order to minimize the visual impact, while increasing their integration in the surrounding landscape, or be equipped with photovoltaic or thermodynamic panels 726, in order to minimize the electric power consumption of the system thus implemented.

The individual plurifunctional modular units present in the selected system are periodically loaded, with cycles variable from 5 to 365 days as a function of the type of insect to be reared, by using machines not described in the present patent application (apart from that illustrated for the trailer of the system in figure 23), with matrices formed of agri-food waste and/or organic waste and, the first day of the cycle only, with a batch of insects being in an immature stage.

Periodically are said modular units loaded with an amount of matrix that is proportional to the development stage of the reared insects.

The process control software identifies the modular units into groups, as many as the days of a treatment cycle.

Internally to the adopted system, every group can be conditioned to a specific temperature by using a properly heating or cooling air stream, depending on the needs identified by the hardware/software device, in order for every modular unit to be kept at a temperature in the range from 15°C to 30°C.

The control software assigns given climatic treatment conditions to every group, functional to the day of the cycle and to the consequent heating or cooling needs.

As a matter of fact, during said cycle, basic differences between one step of the process and another exist, depending on the age of the larvae present inside the component 200 of the modular unit and on the amount of the matrix wherein they are dispersed, as well as on the degree of oxidation thereof and on the development of the bacterial colonies present therein.

As a matter of fact, the first three days of a cycle usually require a minor heating of the component 200 of the modular unit, whereas cooling might be required from the sixth day to the fifteenth day; therefore, the hardware/software device properly controls a latent heat recovery system, based on emitted vapor condensation mechanisms, to subsequently exchange the recovered heat with the incoming fresh air stream. Finally, at the end of a treatment and/or breeding cycle, specifically in the case of a saprophagous dipteran treatment/breeding, the hardware/software device orders that no air stream circulate in those modular units which are in this stage.

In the course of the twenty-four hours subsequent to the instant in time no air stream is allowed to circulate in the modular units as indicated above, the percentage of oxygen drastically lowers inside the components 200 of the modular unit, and a given oxygen gradient is established between said component and the technical compartments present in the component 100 associated therewith, which drives the larvae present in the component 200 to leave it toward an oxygen gradient featuring a higher percentage, by using the migration paths, this way finally they accumulate in the technical compartments present in the component 100.

In order to provide a good quality in the insect breeding and/or organic waste bioconversion and unsorted waste biostabilization process and to certify the products resulting therefrom, the hardware/software device monitors and controls said processes all along the complete production path and possibly uses the blockchain technology to file said information in a safe manner and to generate a traceability that covers every step of the chain, from production to logistics, up to product and byproduct distribution.

Thanks to the Internet of Things (loT) devices integrated in the modular units, tag RFIDs, and hardware/software process control devices, every data item or event acquired in the plurifunctional modular units is univocally logged and validated in real time by the blockchain public ledger, in order for it to become unalterable and shareable by all authorized actors of the chain via computers, tablets, smartphones, or any other means that are capable of accessing the internet network.

In order to reduce the energy costs for maintaining the climatic conditions necessary for the insect breeding and/or organic waste bioconversion and unsorted waste biostabilization process, every system according to the present invention can be provided with a heat recovery means based on condensing thermal exchange mechanisms.

Said heat recovery means comprises a condenser, which is a heat exchanger by means of which the steam coming from the plurifunctional modular units is condensed into a liquid; this takes place thanks to the steam cooling down and a refrigerating fluid heating up, which crosses the heat exchanger and consequently performs the task of subtracting latent heat from the steam to be condensed.

The condensers can have exchange surfaces very variable in geometry and size, depending on the number of plurifunctional modular units that the selected system is equipped with.

The waste air stream output from the condenser can be compressed, for example up to 7 atm, to liquify the ammonia possibly present therein, in order to recover and use it in other production processes.

The scope of this invention is susceptible of numerous modifications and variants, all falling within the inventive concept set forth in the attached claims.

All details might be replaced by other technically equivalent elements, depending on the actual requirements, without departing from the scope of protection of the present invention.

Even though the scope has been described with a particular reference to the attached figures, the reference numerals used in the description and in the claims are used for improving the understandability of the invention and do not constitute any limitations to the claimed scope of protection.

## Claims

1. A plurifunctional modular unit for insect breeding and egg laying, and for agri-food waste and organic waste biotreatment, usable in technological storage and electromechanical handling systems and in standard industrial logistic plants, once modified and implemented with further technologies as necessary for said breeding and egg laying and biotreatment activities, **characterized in that** it comprises the following components combined with each other:
- a base component (100), common to all modular units, also operating as an interface to the systems to handle the same units via storage and electromechanical handling systems;
- a component (200) functional to the breeding and/or agri-food waste and/or organic waste bioconversion and biostabilization processes and alternatively a cell component (400) functional to the adult insect breeding and egg laying processes;
- a first cover component (300) for closing said components (200, 400);
- a second cover component (500) for closing said components (200, 400);
- a third cover component (600) stably connected to the storage and electromechanical handling systems for closing said components (200, 400).

2. The plurifunctional modular unit for insect breeding and for agri-food waste and organic waste bioconversion and biostabilization processes, usable in technological storage and electromechanical handling systems and in standard industrial logistic plants, once modified and implemented with further technologies as necessary for said breeding and/or biotreatment activities, **characterized in that** it comprises the following components:
- a base component (100) also operating as an interface to the systems to handle the same units via storage and electromechanical handling systems;
- a component (200) for breeding and/or agri-food waste and/or organic waste bioconversion and biostabilization;
- a cover component (300, 500) for closing said component (200).

3. The plurifunctional modular unit functional to the adult insect breeding and egg laying processes, usable in technological storage and electromechanical handling systems and in standard industrial logistic plants, once modified and implemented with further technologies as necessary for said breeding and egg laying activities, **characterized in that** it comprises the following components:
- a base component (100) also operating as an interface to the unit systems to handle the same units by means of storage and electromechanical handling systems;
- a component (400) for adult insect breeding and egg laying;
- a cover component (300, 500) for closing said component (400).

4. The plurifunctional modular unit according to any of the previous claims, **characterized in that** the base component (100) comprises a double-walled parallelepiped shaped plastic container, featuring an open upper face, and is provided with:
- protrusions (101) placed at the four corners, operating as feet,
- ribs or stiffeners (102) as necessary to support the full load weight of the components placed above,
- protrusions and/or arrangements (103) on the top at the four sides for coupling with a component placed above by means of joints and/or handles,
- one or more ribs (104) partitioning the inner space into technical compartments provided with exhaust holes and/or holes for interfacing to the outside (105);
- sliding electrical contacts (106, 107) for supplying power to the units whenever they are placed inside the storage and electromechanical handling systems, arranged at the base of at least two feet (101) and in the upper part thereof, each in a diagonally opposed position;
- cylindrical holes (108), provided with a check valve crossed by connectors arranged in the storage and electromechanical handling systems, for the communication between the plurifunctional unit and a capillary climatization plant;
- through holes (109), on the lateral surface of the component (100), for creating an oxygen gradient between the inner space and the outside of the plurifunctional unit;
- RFID means.

5. The plurifunctional modular unit according to claims 1, 2, and 4, **characterized in that** the component (200) for breeding and/or agri-food waste and/or organic waste bioconversion and biostabilization, placed above the base component (100), comprises a double-walled parallelepiped shaped plastic container, featuring an open upper face, and is provided with:
- protrusions and/or arrangements (201) placed on the top and on the bottom at the four sides for coupling with a component placed above and/or below by means of joints and/or handles;
- holes (202, 206) for connecting through cylindrical elements to convey air conditioning into the base component (100) and into the cover component (300, 500);
- a central circular area (203), placed on the bottom of the container and depressed with respect to the point where the bottom itself joins to the sides of the parallelepiped,
- holes (204) close to the lateral walls for liquid percolation;
- trapezoidal shaped protrusions (205), forming migration paths, placed all along the inner walls, alternating to each other at a variable distance, featuring a hole (206), operating as a migration hole, on their top, at the middle of the higher side of the trapezium;
- sliding electrical contacts (207) placed on the lower and upper outer edges, for transmitting or receiving electric power to/from the components (100, 300, 500);
- sliding electrical contacts (208) placed on the sides, for supplying power to the units whenever these are placed inside the storage and electromechanical handling systems;
- metal tracks (209) parallel to each other, made from a conductor material, on the upper inner edge, above the migration holes (206), for routing the electricity propagated by the sliding electrical contacts;
- RFID means.

6. The plurifunctional modular unit according to claims 1, 3, and 4, **characterized in that** the component (400) for adult insect breeding and egg laying, placed above the base component (100), comprises a double-walled parallelepiped shaped plastic container, featuring an open upper face, and is provided with:
- protrusions and/or arrangements (401) placed on the top and on the bottom at the four sides for coupling with a component placed above and/or below by means of joints and/or handles;
- two opposed tilted planes (402), placed on the bottom, which join the lateral surfaces to the ribs (104) of the base component (100);
- compartments (403) accessible from the outside;
- sliding electrical contacts (404), placed on the lower and upper outer edges, for transmitting or receiving electric power to/from the components (100, 300, 500);
- sliding electrical contacts (405), placed at the sides, for supplying power to the units whenever these are placed inside the storage and electromechanical handling systems;
- RFID means.

7. The plurifunctional modular unit according to the previous claims, **characterized in that** the first cover component (300), for closing the components (200, 400), is provided with:
- protrusions and/or arrangements (301) placed at the four sides for coupling with the component (200, 400) placed below;
- lateral connection holes (302);
- lower distribution holes (303);
- an opening (304) for feeding the organic matrix;
- an electromechanically operated hatch (305) for closing the opening (304) of the cover of the component (200);
- a LED lighting panel (306) for closing the opening (304) of the cover of the component (400);
- lower cylindrical holes (307) provided with a check valve and connectors arranged in correspondence with at least two migration holes of the component (200), for the communication between the plurifunctional unit and a capillary climatization plant arranged in the storage and electromechanical handling system;
- depressions (308), placed at every corner, for accommodating and leading-in plurifunctional modular units superimposed on each other;
- sliding electrical contacts (309), on the lower edge, for receiving electric power from the components (200, 400);
- an RFID tracking system.

8. The plurifunctional modular unit according to the previous claims, **characterized in that** the second cover component (500) used for closing the components (200, 400) is provided with:
- protrusions and/or arrangements (501), located at the four sides for coupling with a component (200, 400) placed below;
- an opening (502) for organic matrix supply;
- an electromechanically operated hatch (503) for closing the opening (502) of the cover of the component (200);
- a LED lighting panel (504) for closing the opening (502) of the cover of the component (400);
- an ambient humidification system,
- a first passage (506) for letting air pass from the outside to inside, connected to a box containing one or more Peltier cells,
- a second passage (505) for letting exhausted air pass from the inside to the outside, via an extraction fan;
- a wi-fi antenna (507) or an antenna according to another similar technology for data transmission;
- a set of humidity, temperature, oxygen, etc. control sensors;
- a programable logic controller (PLC) for device control;
- a battery (508);
- sliding electrical contacts (509), on the lower edge, for receiving electric power from the components (200) or (400);
- an RFID tracing system.

9. The plurifunctional modular unit according to the previous claims, **characterized in that** the third cover component (600) for closing the components (200, 400), is provided with:
- holes (601) for air conditioning the modular unit by means of a set of pipes (602);
- lighting means (603);
- pneumatic joints (604) for connecting to the structures of the storage and electromechanical handling systems;
- gaskets (605), placed all along the lower edge, in correspondence with the connection to a modular unit;
- sliding electrical contacts (606), placed on the lower edge, conjugated with the sliding electrical contacts (207, 404) of the components (200, 400) for allowing its electric power supply.

10. The plurifunctional modular unit according to the previous claims, **characterized in that** every component (100, 200, 300, 400, 500, 600) has dimensions in the horizontal plane equal to those of the component placed below/above, which it can be connected to by means of protrusions and/or arrangements for joint and/or handle coupling.

11. The plurifunctional modular unit according to the previous claims, **characterized in that** every component (100, 200, 300, 400, 500, 600) is insulated by insufflation or application of an insulating material.

12. The plurifunctional modular unit according to the previous claims, **characterized in that** it is provided with at least one hardware/software device comprising a central check and control unit connected to peripherals capable of detecting parameters such as temperature, quantity of oxygen, humidity, and pH inside the plurifunctional modular units, storing said parameters in a dedicated server and sending controls aiming at controlling the processes by means of dedicated actuators.

13. A technological system for storing and electromechanically handling at least one plurifunctional modular unit according to anyone of the previous claims, **characterized in that** it comprises the following self-supporting structures provided with specific devices:
- vertical elements (701) provided with uprights and/or shoulders and/or side frames of different heights and depths, structured and possibly latticed,
- girders (702) operating in association with said vertical elements (701) to form rows spaced away from each other, for creating corridors according to an adopted project diagram;
- fixed inter-storey loading levels (703) as a function of the heights available and the maximum loading elevation practicable,
- lifting and handling means (704), such as stackers, front and retractable forklifts, bilateral and trilateral, operator guided or automated guided (e.g., AGV, LGV, IGV, etc.) forklifts provided with appropriate forks;
- sliding and/or direct electrical contacts (705) for any positions reserved for the plurifunctional modular units.

14. The technological system for storing and electromechanically handling at least one plurifunctional modular unit according to anyone of the previous claims, **characterized in that** it comprises the following self-supporting structures provided with specific devices:
- vertical elements (701) provided with uprights and/or shoulders and/or side frames of different heights and depths, structured and possibly latticed, provided with a number of brackets full-height mounted laterally thereto, at a constant distance from each other,
- three-axis modular unit handling systems (706) of a horizontal girder or vertical gantry, or overhead crane or reversed translation type, provided with appropriate winches for picking-up and fast handling of the modular units;
- vertical compartments (707) inside which the modular units are handled;
- chain/roller conveyors (708) for transporting the modular units to a manual, semi-automated, or fully automated station (709), and for their handling or loading, unloading, and/or cyclical control.

15. The technological system for storing and electromechanically handling at least one plurifunctional modular unit according to anyone of the previous claims, **characterized in that** it comprises the following self-supporting structures equipped with specific devices:
- vertical elements (701) provided with uprights and/or shoulders and/or side frames of various heights and depths, structured and possibly latticed, provided with horizontal spacers and/or stringers, which determine points at a fixed height on which plurifunctional modular units featuring the same maximum size can be arranged according to a multi-depth approach (710);
- latticed skids (711);
- an automated machine (712) comprising a trailer, whose minimum front size equals at least that of the skid, for handling, by means of removal, on a double lateral front of the latticed skids (711) loaded with modular units to be performed on board the machine,
- buffer tanks (714) associated with the trailer (712),
- a meter (713) mounted on the trailer which picks up matrices from one or more buffer tanks (714).

16. The technological system for storing and electromechanically handling at least one plurifunctional modular unit according to anyone of the previous claims, **characterized in that** it comprises the following self-supporting structures provided with specific devices:
- horizontal surfaces (715) featuring wide bays and stacked galleries,
- stations arranged according to an electrified grid (716) for placing modular units in a single mode (717) or in a stacked mode (718),
- automatic guided machines (719) of the AGV, LGV, IGV, etc. types, for a cyclical handling and conveyance of the modular units to appropriate areas or conveyors (720),
- elevators (722) for sorting the modular units toward work, test, and/or production bays (721);
- chain and/or roller conveyors (723);
- stacking machines (724).

17. The technological system for storing and electromechanically handling at least one plurifunctional modular unit according to claims 13 to 16, **characterized in that** it is inserted into a casing comprising walls and perimetral coverings (725) formed of:
- insulating panels made from metal, wood and/or technical materials providing high thermal and acoustical insulation and mechanical strength.
- photovoltaic or thermodynamic panels (726).

18. The technological system for storing and electromechanically handling at least one plurifunctional modular unit according to claims 13 to 16, **characterized in that** it is provided with a heat recovery means based on condensing mechanisms having exchange surfaces, variable in geometry and dimensions as a function of the number of plurifunctional modular units that the selected system is provided with.
